# FASCICULE DE BREVET EUROPEEN

(11) **EP 3 595 901 B1**
(45) Date de publication et mention de la délivrance du brevet: **05.05.2021**
(21) Numéro de dépôt: 18712975.4
(22) Date de dépôt: 08.03.2018
(51) Int. Cl.: B41J 2/04, B41J 2/14, B33Y 80/00, C12M 3/00, A61F 2/50

(54) **EQUIPEMENT POUR LE TRANSFERT DE BIO-ENCRE**
VORRICHTUNG ZUR ÜBERTRAGUNG VON BIOTINTE
APPARATUS FOR THE TRANSFER OF BIO-INK

(30) Priorité: 15.03.2017 FR 1752129
(43) Date de publication de la demande: 22.01.2020
(73) Titulaire: Poietis, 33600 Pessac (FR)
(72) Inventeur: VIELLEROBE, Bertrand, 33700 Merignac (FR); VAUCELLE, Romain, 33000 Bordeaux (FR); GUILLEMOT, Fabien, 33210 Preignac (FR)
(74) Mandataire: IP Trust
(86) Numéro de dépôt international: PCT/FR2018/050533
(87) Numéro de publication internationale: WO 2018/167400

(56) Documents cités:
- WO-A1-2016/097619
- WO-A1-2016/097620
- WO-A1-2017/011854

## Description

### Domaine de l'invention

La présente invention concerne le domaine de la bioimpression par laser par un procédé de transfert assisté par ordinateur pour la modélisation et l'assemblage de matériaux vivants et optionnellement non-vivants avec une organisation prescrite 2D ou 3D dans le but de produire des structures de bioingénierie servant en médecine régénérative, en pharmacologie et pour des études de biologie cellulaire.

La bioimpression assistée par laser permet d'organiser avec une haute précision les éléments individuels du tissu pendant sa fabrication via le dépôt couche après couche de cellules et de biomatériaux. Elle permet de reproduire des tissus en 3D avec une géométrie spécifique. L'approche « bottom-up », basée sur l'assemblage brique par brique puis couche par couche d'un objet, est compatible avec une automatisation du procédé de fabrication du tissu et peut fonctionner dans un environnement stérile. De plus, l'automatisation pourrait permettre de réduire les coûts, d'améliorer la qualité et la reproductibilité de la fabrication de tissus biologiques.

L'invention concerne plus particulièrement une solution de dépôt assisté par laser basée sur l'action directe (absorption de la radiation laser) et indirecte (création d'un plasma et d'une bulle de cavitation) d'un faisceau laser pour diriger le dépôt de particules vers un substrat d'impression avec une résolution micrométrique.

### Etat de la technique

L'article « F. Guillemot, A. Souquet, S. Catros, B. Guillotin, J. Lopez, M. Faucon, B. Pippenger, R. Bareille, M. Rémy, S. Bellance, P. Chabassier, J. C. Fricain, and J. Amédée, "High- throughput laser printing of cells and biomaterials for tissue engineering," Acta Biomater. 6, 2494-2500 (2010) » décrit un exemple d'équipement pour mettre en œuvre un tel procédé.

On connaît aussi dans l'état de la technique les demandes de brevet WO2016097619 et WO2016097620 décrivant un procédé et un équipement d'impression d'au moins une encre, ledit procédé comprenant une étape de focalisation d'un faisceau laser de manière à générer une bulle de cavitation dans un film d'encre, une étape de formation d'au moins une gouttelette d'encre à partir d'une surface libre du film d'encre et une étape de dépôt de ladite gouttelette sur une surface de dépose d'un substrat receveur, caractérisé en ce que le faisceau laser est orienté à contresens par rapport à la force gravitationnelle, la surface libre du film étant orientée vers le haut en direction de la surface de dépose placée au-dessus du film d'encre.

Cette configuration permet notamment d'obtenir une épaisseur E pour le film d'encre sensiblement constante, tout en limitant l'apparition des phénomènes de sédimentation. De plus, elle permet d'utiliser une large gamme d'encres. Cette configuration permet également d'utiliser des moyens d'imagerie résolue en temps (TRI) par ombroscopie pour étudier et contrôler la formation des jets.

Un autre exemple de technique d'imagerie temps réel appliquée au suivi de l'ablation laser de neurones est décrit dans la publication «Real time imaging of femtosecond laser induced nano-neurosurgery dynamics in C. elegans », OPTICS EXPRESS 364, Vol. 18, No. 1, 4 January 2010. Dans cette configuration, le champ de vue est très petit et centré sur la zone d'ablation laser où un certain nombre de phénomènes photochimiques et photo-biologiques complexes sont étudiés. Il s'agit d'un exemple général d'imagerie associée à un processus d'interaction laser-matière pour du micro-usinage.

### Problème technique de l'art antérieur

Dans le domaine de la bio-impression, les bio-encres sont des milieux dits inhomogènes, c'est à dire qu'elles contiennent soit des particules en suspension, soit différentes espèces biochimiques en solution (facteurs de croissance, molécules biologiques, ions, etc...) soit encore des biomatériaux (répartis en solution de façon uniforme, ou de gradient ou de flux colinéaires s'il y en plusieurs), soit un mélange plus ou moins complexe de ces trois principaux constituants, ce dernier cas étant le plus commun. La complexité de ces encres, en particulier leur caractère inhomogène n'est pas du tout traité dans l'art antérieur de la bio-impression vers l'avant par laser.

De plus, l'art antérieur a démontré que la densité de particules d'une bio-encre était fortement aléatoire (fluide colloïdal), conduisant ainsi à un transfert d'un nombre de particules vers un substrat récepteur de façon statistique.

Dans un tel contexte, les solutions de l'art antérieur ne permettent pas de sélectionner dans le film d'une bio-encre une composition précise (particules ou espèces biochimiques ou biomatériaux), en fonction de caractéristiques particulières la distinguant d'autres éléments composants la bio-encre présents dans le film, pour transférer sur le récepteur la bonne composition de bio-encre correspondant au plan de bio-impression prédéfini.

Dans les solutions de l'art antérieur, le laser interagit avec une zone du film supposée contenir des inhomogénéités à transférer, « à l'aveugle », le film contenant une densité de particules transférables suffisante, voire proche de la saturation pour garantir le transfert d'un nombre suffisant de particules.

Le film est rechargé périodiquement de manière manuelle avec un fluide contenant de nouvelles particules, de façon à maintenir un nombre de particules suffisant, permettant un transfert à l'aveugle. On peut souligner qu'une telle problématique est finalement commune à tous les procédés de bio-impression, notamment le jet d'encre et l'extrusion.

### Solution apportée par l'invention

En préambule, on entend par « inhomogénéité » du film de bio-encre, toute zone du film ayant des caractéristiques propres en terme de composition : soit de particules, soit d'espèces bio-chimiques (facteurs de croissance, molécules biologiques, ions, etc.) soit encore de biomatériaux. De façon générale, les termes « zone inhomogène », « variation locale de composition », « zone de composition spécifique » sont utilisés comme synonymes du terme générique « inhonomogénéité ».

Afin de répondre à ces inconvénients, l'invention concerne selon son acception la plus générale un équipement pour le transfert d'une bio-encre sur une cible comportant :
- une lame transparente définissant une zone de réception d'un film de fluide hétérogène contenant soit une pluralité de particules et / ou une pluralité d'espèces biochimiques et /ou une pluralité de biomatériaux, soit une pluralité de particules et / ou un ou plusieurs types biomatériaux et / ou une ou plusieurs espèces bio-chimiques et / ou un ou plusieurs types d'espèces biochimiques
- une source laser associée à un moyen de déviation piloté et un bloc optique de focalisation dans un plan du film de fluide pour appliquer une impulsion localisée
caractérisé en ce que
ledit équipement comporte en outre
- un moyen d'imagerie d'une zone d'imagerie présentant une section d'imagerie au moins 5 fois supérieure à la section nominale d'une inhomogénéité du film de bio-encre
- des moyens d'analyse des images pour reconnaître
   ∘ les positions géométriques des inhomogénéités dans le film
   ∘ une caractéristique observable de chacune des inhomogénéités reconnues (taille, facteur de forme, type de particules, âge de la particule, densité et type du biomatériau, molécules,...) par des moyens de caractérisation (comme par exemple l'imagerie optique, la spectroscopie Raman ou encore l'analyse de l'auto-fluorescence) et par des moyens d'analyse spatio-temporelle (dynamique du comportement individuel et / ou de groupe d'inhomogénéités, etc.) et
- des moyens de sélection d'au moins une des inhomogénéités ainsi localisée et caractérisée, et de commande du moyen de déviation pour diriger le faisceau laser vers la position de l'inhomogénéité et déclencher le tir laser.

Selon certaines de ses variantes :
- le champ d'imagerie supérieur est à 1 x 1 millimètre
- le champ d'imagerie est modulable par application d'une ROI (région d'intérêt)
- la zone d'imagerie comprend la zone d'interaction du laser avec le film de fluide
- la zone d'imagerie est distincte de la zone d'interaction du laser avec le film de fluide, par exemple sur 2 plans focaux distincts ou sur deux zones adjacentes d'un même plan focal.
- l'équipement comporte un calculateur local exécutant des traitements d'image pour la caractérisation des inhomogénéités présentes dans la zone d'imagerie.
- l'équipement comporte des moyens de communication avec un calculateur distant exécutant des traitements d'images pour la caractérisation des inhomogénéités présentes dans la zone d'imagerie.
- les moyens de caractérisation comprennent un ou plusieurs des éléments suivants : un système d'imagerie optique mono ou multi-chromatique, un spectromètre Raman, un spectromètre InfraRouge ou encore un moyen d'analyse de l'auto-fluorescence endogène des particules et des biomatériaux.
- le film contient des cellules vivantes, procaryotes ou eucaryotes, en suspension ou agrégées.
- Le film contient un ou plusieurs biomatériaux qui peuvent être potentiellement mélangés avec des cellules d'un ou plusieurs types ainsi qu'à des espèces biochimiques telles que des facteurs de croissance ou certaines biomolécules. Une telle configuration ouvre la voie à la constitution d'encres très complexes et hétérogènes qui pourront être utilisées comme encre « unique » pour réaliser certains tissus en simplifiant le procédé et le rendant plus rapide et moins coûteux. La complexité résidera alors dans la capacité à la présente invention de traiter rapidement la localisation et la caractérisation de multiples zones de particules, biomatériaux et / ou espèces chimiques présentes dans le film de fluide pour permettre la fabrication maîtrisée de tissus complexes et personnalisables.
- Le système pourra également être avantageusement associé à un dispositif de rechargement continu en bio-encre afin de garantir une bonne productivité tout donnant accès à une large gamme d'inhomogénéités à cibler pour répondre au plan de bio-impression prédéfini.

L'invention concerne aussi un procédé pour le transfert d'une bio-encre comprenant des inhomogénéités sur une cible consistant à disposer dans un équipement de transfert une lame transparente définissant une zone de réception d'un film de fluide transparent contenant une pluralité d'inhomogénéités (particules ou espèces biochimiques ou biomatériaux) et commander l'orientation et le déclenchement d'une impulsion laser émise par un laser associée à un moyen de déviation piloté, pour provoquer une interaction du faisceau laser avec ledit film,
caractérisé en ce que

le procédé comporte entre outre :
a) au moins une étape d'imagerie d'une zone du film présentant une section d'imagerie au moins 5 fois supérieure à la section nominale d'une inhomogénéité
b) au moins une étape d'analyse des images pour reconnaître les positions géométriques des inhomogénéités et au moins une caractéristique observable de chacune des inhomogénéités reconnues (taille, facteur de forme, type de particules, âge de la particule, type et densité de biomatériau, molécule,...) et
c) au moins une étape de sélection de l'une des inhomogénéités ainsi localisée et caractérisée, et
d) au moins une étape de transfert comprenant une commande du moyen de déviation pour diriger le faisceau laser vers la position de l'inhomogénéité sélectionnée et une commande de déclenchement d'un tir laser.

Selon des variantes du procédé :
- il comporte en outre une étape d'imagerie, d'analyse et de caractérisation et une pluralité d'étapes de transfert correspondant chacune à au moins une zone à transférer localisée et caractérisée.
- il comporte alternance d'étapes d'imagerie, d'analyse et de caractérisation d'une part, et d'étapes de transfert d'autre part.
- il comporte une étape de calcul d'une séquence ordonnée de zones à transférer.
- l'étape d'imagerie comporte des traitements des images brutes pour extraire des motifs simplifiés correspondant aux zones à transférer.
- les étapes de calcul de position et de caractérisation des zones à transférer sont réalisées simultanément.
- les étapes de calcul de position et de caractérisation des zones à transférer sont réalisées par des traitements basés sur des algorithmes exécutés sur des processeurs à architecture parallèle.
- il comporte une étape additionnelle de comparaison d'une image de la zone imprimée après un transfert, et une carte des implantations théoriques.
- il comporte une étape additionnelle de déclenchement d'un nouveau transfert pour corriger la différence entre le transfert observé et l'implantation théorique.
- ledit film contient des cellules vivantes,
- ledit film contient un ou plusieurs types d'espèces biochimiques,
- ledit film contient un ou plusieurs types de biomatériaux,
- ledit film contient des concentrations différentes d'un même biomatériau,
- le procédé peut comprendre également des moyens de rechargement du film de bio-encre permettant de remplir de façon continue ou discontinue la zone des tirs lasers. Les inhomogénéités de la bio-encre sont alors forcément en mouvement au sein du film, ce qui implique l'utilisation de moyens adaptés d'acquisition et de traitement de données pour caractériser la bio-encre de façon rapide et certaine. Ces moyens peuvent couvrir le recours à l'imagerie, la spectroscopie, l'analyse physico-chimique, la mesure en ligne, etc.

On entend par « inhomogénéité » au sens de la présente invention une zone d'intérêt de la bio-encre de composition organique, minérale ou vivante, en particulier :
- des particules nanoscopiques telles que des exosomes ou autres vésicules produites par les cellules ou des nanoparticules de biomatériaux (hydroxyapatite) ou encore des nano-capsules de biomolécules (facteurs de croissance),
- des particules microscopiques telles que des cellules vivantes (cellules eucaryotes, cellules souches, globules,...), des micro-particules de biomatériaux, des micro-capsules de biomolécules (facteurs de croissance),
- des particules mésoscopiques telles que des sphéroïdes constitués par des amas de cellules ou de biomatériaux.

De préférence, dans le contexte de la bio-impression, on entend par particules, des objets ayant des propriétés biologiques, comme par exemple, des cellules vivantes, des exosomes ou encore des biomolécules.

Mais toutefois, cet équipement et le procédé correspondant ne s'arrêtent pas à cette définition selon l'invention. En effet, les particules peuvent également être non biologiques (c'est à dire inertes) et constituées par exemple d'un ou plusieurs biomatériaux, leur nature dépendant de l'application visée.

### Description détaillée d'un exemple non limitatif de réalisation

D'autres caractéristiques et avantages ressortiront de la description qui va suivre de l'invention, description donnée à titre d'exemple uniquement, se référant aux dessins annexés sur lesquels :

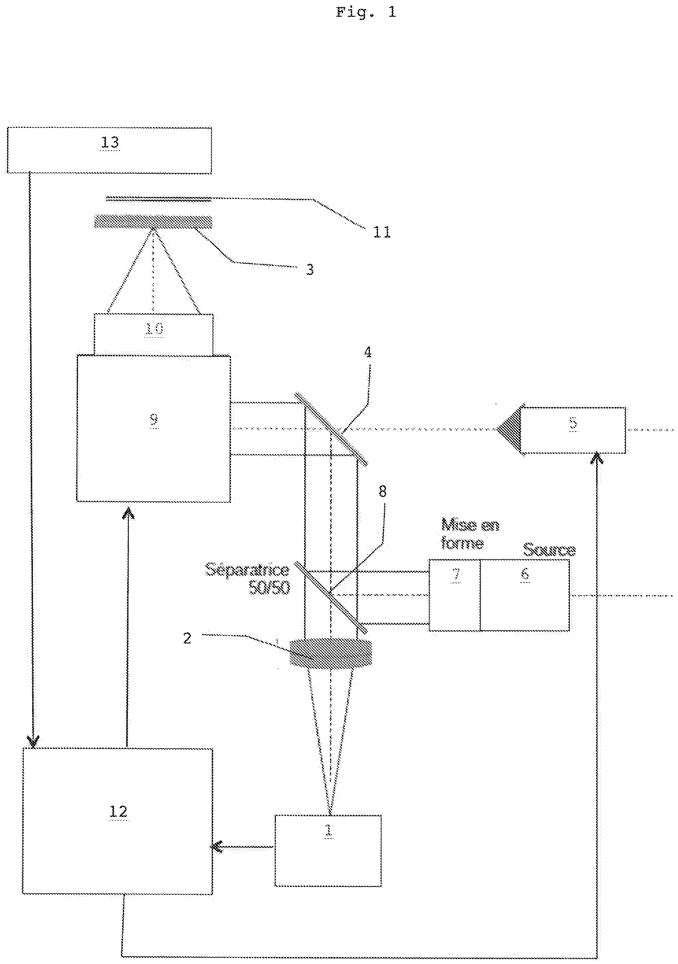

- La figure 1 représente une vue schématique d'un équipement selon l'invention.
- La figure 2 représente des vues des images acquises.

### Contexte de l'invention

L'invention s'inscrit dans le domaine de la bio-impression par laser (LAB) qui a pour but de reconstruire des tissus humains en 3D. Le principe du LAB consiste à focaliser un laser pour créer un plasma par absorption sur un film de bio-encre constitué d'une solution de biomatériaux, d'espèces biochimiques et / ou d'une suspension cellulaire dans un milieu liquide. A partir de ce plasma, une bulle de cavitation est générée dans la bio-encre. Cette bulle par son mouvement hydrodynamique permet de déformer la surface libre de la bio-encre jusqu'à créer un jet de matière. Les caractéristiques de ce jet dépendent d'un grand nombre de paramètres physiques. C'est par ce jet (qui contient un petit nombre de cellules, biomatériaux ou espèces chimiques) que le transfert de matière au substrat receveur s'opère de façon contrôlée.

Dans ce contexte, il convient d'imprimer les constituants des tissus biologiques selon des motifs (patterns) / des localisations bien précises afin d'obtenir
- soit des objets 2D / 3D qui auront des propriétés (formes et fonctions) les plus proches possibles des tissus vivants natifs
- soit des chimères permettant de complexifier / tester / simuler des contextes biologiques pour améliorer la compréhension des mécanismes de morphogénèse tissulaire ou de réponse biologique à des agents extérieurs (agents actifs).

Ainsi, l'impression de biomatériaux réalisée conjointement à celle des cellules doit également suivre des schémas d'impression bien spécifiques afin de donner un environnement viable aux cellules imprimées pour réaliser l'objet demandé. En résumé, la maîtrise de la quantité et de la position des cellules / biomatériaux / espèces biochimiques imprimés sur le substrat receveur est primordiale pour atteindre la qualité nécessaire et attendue des objets bio-imprimés.

Le but de la présente invention est de mettre en place un moyen de mesure permettant de repérer / cartographier les zones de composition spécifique dans une bio-encre inhomogène avant leur impression et ainsi intimer au laser l'ordre de tirer spécifiquement sur une zone de composition souhaitée en fonction de la zone à imprimer (sur le « receveur ») et du motif conçu lors de la CAO de l'objet cible.

A cet effet, l'invention consiste à :
i) à réaliser une caractérisation 2D du donneur
ii) à détecter les inhomogénéités par un traitement numérique ad-hoc
iii) à cartographier automatiquement les positions des zones de composition spécifique
iv) à faire correspondre le schéma des tirs lasers (trajectoire d'impression) avec la cartographie des zones de composition spécifique du donneur

Dans le contexte de la bio-impression, plus particulièrement lors de la mise au point de nouveaux modèles, des marqueurs peuvent être utilisés de façon temporaire pour taguer les cellules par des agents extérieurs (immuno-marquage, fluorescence...) afin d'aider à la caractérisation des objets imprimés. Par contre, lors de la production de modèles qu'ils soient destinés à des applications in vitro ou in vivo, les cellules doivent rester totalement intègres de toutes perturbations exogènes car elles constituent les briques de base du tissu cible. Elles ne pourront donc pas être marquées par des agents extérieurs dans ces cas là. Ainsi, la détection des cellules sera relativement compliquée au sein des milieux de culture car leur indice de réfraction est assez proche de celui de l'eau, principal constituant de ces milieux. L'imagerie cellulaire présente donc déjà un problème important à relever.

Par ailleurs, la dynamique de remplissage de la tête d'impression ou encore le mouvement naturel des cellules en suspension ont une incidence directe sur la position des cellules dans le temps. Cela implique de détecter leur position de façon répétée et à haute fréquence si on veut que la trajectoire d'impression laser soit toujours en accord avec la cartographie de leur localisation. La conséquence est directe sur les moyens de détection et de cartographie qui doivent fonctionner à des cadences élevées (à la fois pour la partie hardware permettant l'acquisition des données et pour la partie software permettant le traitement des données).

Le problème posé et que l'invention vise à résoudre est donc multiple :
- détecter des variations locales de composition des bio-encres (inhomogénéités) qui sont difficiles à visualiser (moyens d'imagerie + traitement d'image),
- cartographier les inhomogénéités (traitement d'image permettant de donner à la fois leur nombre et leur position dans le donneur)
- Effectuer les tirs lasers en correspondance avec la cartographie (adaptation de la trajectoire d'impression en temps réel)

Selon une variante, cette invention vise également à couvrir des cas très intéressants de tri cellulaire :
- être capable de distinguer entre cellules vivantes et cellules mortes afin de n'imprimer que des cellules vivantes
- être capable de dissocier deux types cellulaires qui auraient étés mélangés (volontairement par co-culture ou non) dans une même encre
- être capable de distinguer des cellules d'un même type mais présentant une forte disparité (cellules souches, différents niveaux de différentiation / maturité)
- être capable de distinguer des zones de concentrations d'un même biomatériau qui seraient présentes dans la zone d'observation et produites volontairement ou non
- être capable de distinguer différents biomatériaux qui auraient été mélangés dans une même bio-encre.

Au niveau performance, la fonction « Visé-Tiré » objet de l'invention doit permettre de:
- rationaliser le processus d'impression (ratio du nombre de cellules imprimées sur le nombre de cellules présentes dans la bioencre), autrement appelé « Printing Yield ». Cela revient à minimiser le nombre de cellules non utilisées pendant le process.
- optimiser le processus en choisissant les zones de tir laser en fonction du nombre de cellules présentes spatialement (pas de recours à de multiples tirs laser pour imprimer une même zone). Cela revient à optimiser la trajectoire d'impression pour minimiser le nombre de tirs lasers et ainsi minimiser le stress cellulaire.
- accélérer le processus (si besoin) grâce à l'efficacité des tirs laser, autrement appelé « Printing Rate »
- rendre compatible le procédé avec une cartouche à rechargement continu puisque la méthode « Visé-Tiré » doit permettre à tout moment de mesurer les déplacements des cellules dans la bio-encre où l'écoulement sera relativement important.

Les enjeux sont aussi :
- de rendre possible l'impression de cellules en petites quantité ou rares grâce à la maîtrise du « Printing Yield ». C'est un point extrêmement important par rapport à certaines applications en pharmaceutique, cosmétologie ou clinique où le nombre de cellules disponibles est très faible.
- de rendre possible une customisation très poussée des modèles tissulaires par le choix contrôlé des zones de tir laser en fonction du nombre de cellules présentes spatialement. Ainsi, la réponse à des demandes de mise au point de tissus à façon de la part de laboratoires ou d'industriels sera réalisable avec une palette très large de marchés et d'applications, rendue possible par la haute résolution et la haute précision du processus laser assisté par le Visé-Tiré.
- d'ouvrir la porte à l'industrialisation du procédé et à l'augmentation de sa productivité grâce à l'accélération du processus (« Printing Rate ») par l'efficacité systématique des tirs laser assurée par la fonction Visé - Tiré
- de rendre l'utilisation de la technologie beaucoup plus simple car la manipulation des bio-encres sera optimisée et automatisée avec la cartouche à rechargement continu
- de contrôler le nombre de particules transférées à tout moment pendant le processus de bio-impression (comparaison pré-tir laser / post-tir laser) pour assurer une bonne adéquation entre le motif imprimé et le motif conçu par CAO.
- de réduire le coût des dispositifs de bio-impression en intégrant une seule cartouche (ou un nombre réduit de cartouches) dans laquelle seraient placés les différents constituants du tissu qui seraient triés au moment du transfert. Potentiellement, la réduction de coût pourrait même ouvrir la voie à la définition d'une cartouche à usage unique qui serait d'un grand bénéfice pour l'utilisation de la bio-impression dans le domaine clinique.

### Description de l'équipement

Il comprend une caméra (1) constituée par un capteur de haute définition, par exemple de 18 millions de pixels. A titre d'exemple, la caméra (1) est un capteur commercialisé sous la référence « USB 3 uEye CP » par la société IDS.

Cette caméra (1) est associée à un deuxième bloc optique de conjugaison d'image (2) agissant comme une lentille de champ et assurant ainsi la conjugaison de l'image entre le plan focal du film (3) et le plan du capteur de la caméra (1).

Le film (3) est disposé en face d'une cible (11) vers laquelle sont transférées les cellules ou particules, lors du déclenchement d'une impulsion laser.

A titre d'exemple, le deuxième bloc optique de conjugaison d'image (2) est constitué par un objectif, de préférence télécentrique, comprenant au moins 2 lentilles optimisées dans le domaine du visible.

Le trajet optique est renvoyé par une lame dichroïque (4) passe-haut, transmettant l'infrarouge (correspondant à la longueur d'onde d'émission du laser (5)) et réfléchissant les longueurs d'onde dans le domaine du visible.

L'équipement comporte aussi une source d'éclairage (6) émettant dans le domaine du visible associée à une optique de mise en forme (7) dont la fonction est de collimater la source d'éclairage (6) si nécessaire, par exemple lorsque la source présente une divergence du faisceau émis. Cette source d'éclairage peut être une source LED unique, un composant constitué par un assemblage de LEDs, ou une source de lumière blanche type lampes à incandescence, lampes halogènes, laser supercontinuum.... Elle peut aussi être constituée par une source de spectre étroit (soit par la nature même de la technologie employée, soit par l'emploi de filtres optiques) qui émet dans les longueurs d'onde permettant l'excitation de la fluorescence de marqueurs ou de particules.

Une lame séparatrice (8) a pour fonction de superposer le chemin optique d'éclairage et le chemin optique d'observation.

En sortie de la lame dichroïque (4), les deux faisceaux se dirigeant vers le scanner (éclairage et laser) sont colinéaires entre eux et sont de fait aussi colinéaires avec le faisceau d'imagerie revenant du film. Ainsi, les trois faisceaux sont colinéaires entre la lame dichroïque (4) et le film d'encre (3).

Ils sont défléchis par un scanner (9) assurant une orientation pilotée par un calculateur extérieur.

Le scanner (9) assure une orientation angulaire des trois faisceaux colinéaires susvisés, selon deux axes perpendiculaires, 2 d'entre eux étant balayés sur le donneur contenant la bio-encre et le dernier étant « débalayé » en un faisceau d'imagerie collimaté vers le système d'imagerie. Il est par exemple constitué par deux miroirs actionnés par un actionneur électromagnétique, par exemple un scanner commercialisé par la société SCANLAB (nom commercial) sous la référence « SCANcube 14 ».

Les trois faisceaux d'observation, d'éclairage et laser sont ainsi colinéaires et orientés selon une même direction à la sortie du scanner (9). Ainsi, la direction d'observation et la direction d'éclairage suivent l'orientation du faisceau laser.

Le bloc optique (10) a pour fonction :
a) de transformer l'orientation angulaire en un positionnement / déplacement latéral selon deux axes X,Y dans le plan du film (3),
b) de focaliser le faisceau laser et le faisceau d'éclairage dans le même plan du film (3) et
c) de collecter la lumière dans le domaine visible renvoyée par le film (3), pour construire l'image d'observation par la caméra (1).

Le bloc optique (10) est constitué par un ensemble de lentilles formant un objectif télécentrique présentant les caractéristiques suivantes :
Dans le domaine de l'infrarouge, les surfaces des lentilles sont traitées par des couches anti-reflets pour supporter des énergies laser élevées. Cela permet d' éviter la détérioration dans le temps du premier bloc optique (10) dont le design est d'ailleurs calculé pour empêcher la création de points « chauds » laser au sein dudit premier bloc optique (10)

Le miroir dichroïque (4) empêche le retour du rayonnement infrarouge laser vers la caméra (1), lors du déclenchement d'une impulsion. Optionnellement, un filtre réjecteur infra-rouge peut en outre être disposé dans le chemin optique, entre le filtre dichroïque (4) et la caméra (1).

Le rapport de la focale du deuxième bloc optique (2) et de la focale de l'objectif (10) est déterminé pour produire dans le plan de la caméra (1) une image dont les plus petits objets observés présentent une taille de plus d'un pixel.

L'équipement comporte en outre un calculateur (12) recevant les données provenant de la caméra (1) ainsi que d'une seconde caméra (13) observant la cible (11). Ce calculateur (12) pilote également le scanner (9) ainsi que le laser (5).

### Fonctionnement

Le calculateur (12) exécute des programmes d'ordinateur pour effectuer différents traitements :
- un prétraitement des images brutes provenant de la caméra (1)
- un traitement de localisation et de caractérisation des particules identifiées lors du prétraitement précité
- un traitement de pilotage du scanner (9) et du laser (5) permettant de faire correspondre les tirs de celui-ci avec la position des particules
- optionnellement un traitement des images brutes provenant de la caméra (13) observant la cible (11).

### Prétraitement des images brutes provenant de la caméra

Ce prétraitement peut être réalisé périodiquement, pour enregistrer une cartographie du film (3) avant l'engagement d'une séquence de tirs laser, ou entre deux séquences de tirs consécutives.

La première solution est particulièrement adaptée à des situations où le film porte des particules stables, tant en ce qui concerne leur localisation dans le plan du film qu'en ce qui concerne leur évolution. Cela concerne par exemple des particules minérales ou organiques peu réactives par rapport au substrat.

La seconde solution est plus adaptée à des situations où les particules sont mobiles et évolutives. C'est le cas par exemple de particules vivantes telles que des cellules, qui peuvent se déplacer dans le film avec une tendance forte à l'agrégation qui dépendra fortement du type cellulaire et du milieu utilisés.

Dans les deux cas, le traitement consiste à partir des images brutes à extraire des informations correspondant à la détection des objets graphiques correspondant à des particules d'intérêt, par exemple par des techniques de segmentation, de reconnaissance de forme, ou de reconnaissance de contour et de centre de gravité.

Des techniques de seuillage de type watershed, Meyer's flooding algorithm ou d'Optimal spanning forest algorithms peuvent être utilisées pout réaliser cette cartographie.

Ce traitement peut être divisé en plusieurs phases :
- une phase de restauration ou de conditionnement des images brutes (débruitage, défloutage, correction de l'éclairage non uniforme),
- une phase de segmentation
- une phase d'extraction de caractéristiques
- et une phase d'analyse de données.

Ce traitement attribue à chaque objet graphique identifié un identifiant IDᵢ et une position sous forme de coordonnées cartésiennes (Xᵢ,Yᵢ) dans le plan du film.

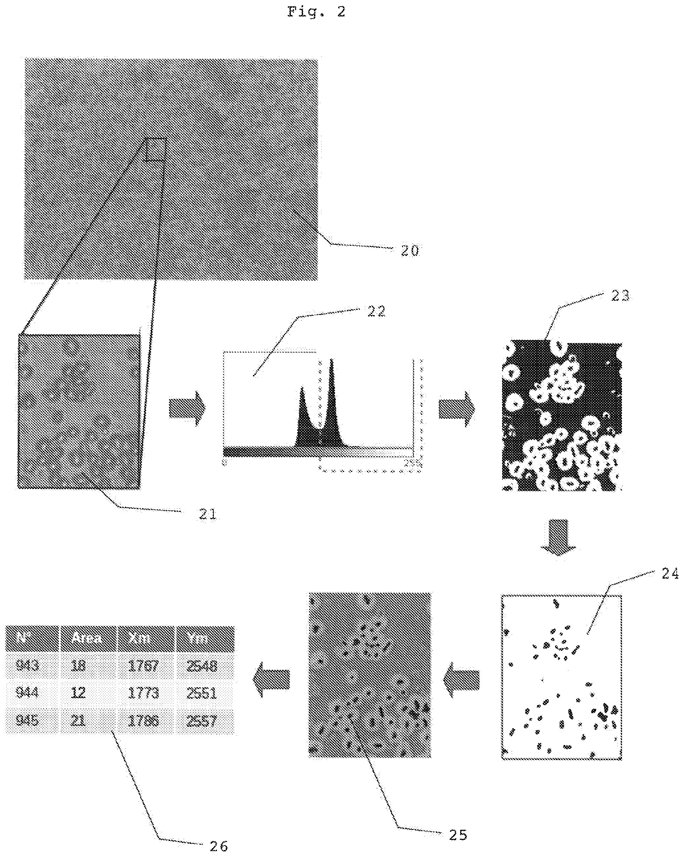

La figure 2 représente la succession d'images, à partir de l'image brute (20) captée par la caméra (1), et d'une zone agrandie (21) montrant la présence d'une pluralité de particules dans le champ de l'image.

On procède de façon connue à un traitement par seuillage pour calculer une variation de contraste sous forme d'histogramme comme illustré par l'image (22). Cet histogramme permet de calculer une image contrastée (23) par des algorithmes de seuillage.

Cette image contrastée (23) permet de calculer les centroïdes (image (24)) et les contours (image (25)).

Ces informations permettent de construire la table (26) des identifiants de chaque objet graphique traité et des coordonnées du centre de chacun de ces objets.

Le traitement de caractérisation consiste à affecter à chacun des objets graphiques identifiés et localisés des attributs en fonction de l'appartenance à une famille prédéfinie par ces paramètres physiques tels que :
- la classe taille de l'objet
- la réaction à un éclairage spécifique, par exemple par photoluminescence
- les caractéristiques spectrales
- le type de contour (forme, régularité,...)
- l'appartenance à un agrégat d'objets
- la densité optique de l'objet
- la densité de l'agrégat (nombre estimé d'objets agrégés).
- la dynamique spatio-temporelle
- ...

Ces paramètres ne sont bien sur pas limitatifs.

Le résultat de ce traitement vient compléter la table susvisée en ajoutant pour chaque identifiant des informations d'appartenance à une ou plusieurs séries de classes.

Les traitements sont de préférence parallélisés et exécutés sur des processeurs à architecture parallèle, notamment GPU.

Les informations sont ensuite exploitées pour mettre en correspondance la cartographie ainsi réalisée, et la carte-cible préalablement enregistrée, de façon à calculer une séquence de tirs laser se traduisant par le calcul d'une orientation du scanner puis le déclenchement de l'impulsion laser, sous la commande du calculateur (12). Le calcul de la séquence prend en compte un calcul de minoration du temps de process global, par des algorithmes connus de type «résolution du problème d'optimisation du voyageur de commerce ou problème NP-complet».

Après chaque tir laser ou après une série de tirs laser, le calculateur vérifie la conformité des transferts par une comparaison entre l'image transmise par la caméra (13) observant la cible (11) et la carte cible préenregistrée. Le calculateur procède à un calcul de maximum de vraisemblance, et en cas de discordance (manque d'objets d'imprimés), recalcule la séquence suivante de tirs laser afin de corriger les anomalies observées. Il peut également permettre de procéder à une correction des zones où il y aurait eu trop d'objets imprimés par rapport à la carte cible. Cette correction se fait généralement par des moyens conventionnels d'aspiration qui permettent d'enlever de façon très précise des petites quantités de matière. Cette aspiration est pilotée par le calculateur (12). Dans tous les cas, on entend ici par pilotage la mise en place d'une boucle d'asservissement entre la carte cible préenregistrée et la cible (11) réellement imprimée qui doit permettre d'obtenir un maximum de vraisemblance le plus proche possible de 100% entre les deux cartes.

En outre, la présente invention ne se limite pas à un seul processus d'impression par laser. Elle couvre en effet l'impression multi-tête (plusieurs films (3) utilisés au même moment avec un ou plusieurs laser), pour laquelle il est impératif que les moyens d'imagerie et leur traitement associé soient capables de traiter plusieurs zones simultanément afin de garantir la possibilité de faire de l'impression « multicouleur » simultanée (plusieurs types cellulaires différents imprimés dans un seul processus).

## Revendications

1. Equipement pour le transfert de bio-encre sur une cible comportant :
- une lame transparente dans le domaine spectral de l'imagerie et du laser (5) définissant une zone de réception d'un film (3) de fluide contenant des hétérogénéités
- une source laser (5) associée à un moyen de déviation piloté et un bloc optique (10) de focalisation dans un plan du film (3) de fluide pour appliquer une impulsion localisée
**caractérisé en ce que**
ledit équipement comporte en outre
- un moyen d'imagerie d'une zone d'imagerie présentant une section d'imagerie au moins 5 fois supérieure à la section nominale d'une inhomogénéité
- des moyens d'analyse des images pour reconnaître
o les positions géométriques des inhomogénéités dans le film, et
o une caractéristique observable de chacune des inhomogénéités reconnues par exemple taille facteur de forme, type de particules, âge de la particule, densité, type de biomatériau, molécule par des moyens d'analyse appropriés, et
- des moyens de sélection d'au moins une des zones inhomogènes ainsi localisée et **caractérisée**, et de commande du moyen de déviation pour diriger le faisceau laser vers la position de la dite zone inhomogène et déclencher le tir laser.

2. Equipement pour le transfert de bio-encre sur une cible selon la revendication 1 **caractérisé en ce que** l'inhomogénéité de la dite bio-encre est constituée de particules.

3. Equipement pour le transfert de bio-encre sur une cible selon la revendication 1 **caractérisé en ce que** l'inhomogénéité de la dite bio-encre est constituée de biomatériaux.

4. Equipement pour le transfert de bio-encre sur une cible selon la revendication 1 **caractérisé en ce que** l'inhomogénéité de la dite bio-encre est constituée d'espèces biochimiques.

5. Equipement pour le transfert de bio-encre sur une cible selon la revendication 1 **caractérisé en ce que** l'inhomogénéité de la dite bio-encre est constituée d'une combinaison de articules, et/ou biomatériaux et/ou espèces biochimiques.

6. Equipement pour le transfert de bio-encre sur une cible selon la revendication 1 **caractérisé en ce que** ladite zone d'imagerie comprend la zone d'interaction du laser avec le film de fluide.

7. Equipement pour le transfert de bio-encre sur une cible selon la revendication 1 **caractérisé en ce que** ladite zone d'imagerie est distincte de la zone d'interaction du laser avec le film de fluide.

8. Equipement pour le transfert de bio-encre sur une cible selon la revendication 1 **caractérisé en ce qu'**il comporte un calculateur local exécutant des traitements d'image pour la caractérisation des inhomogénéités présentes dans la zone d'imagerie.

9. Equipement pour le transfert de bio-encre sur une cible selon la revendication 1 **caractérisé en ce qu'**il comporte des moyens de communication avec un calculateur distant exécutant des traitements d'image pour la caractérisation des inhomogénéités présentes dans la zone d'imagerie.

10. Equipement selon la revendication 1 **caractérisé en ce que** ledit film contient des cellules vivantes.

11. Equipement selon la revendication 1 **caractérisé en ce qu'**il comporte plusieurs zones d'interaction laser - matière.

12. Equipement selon la revendication 1 **caractérisé en ce qu'**il comporte un système d'aspiration pour retirer de la zone d'impression des éléments imprimés en trop.

13. Procédé pour le transfert de bioencre sur une cible consistant à disposer dans un équipement de transfert une lame transparente définissant une zone de réception d'un film de fluide transparent contenant une pluralité d'inhomogénéités, et commander l'orientation et le déclenchement d'une impulsion laser émise par un laser associée à un moyen de déviation piloté, pour provoquer une interaction du faisceau laser avec ledit film,
**caractérisé en ce que**
le procédé comporte entre outre :
e) au moins une étape d'imagerie d'une zone du film présentant une section d'imagerie au moins 5 fois supérieure à la section nominale d'une inhomogénéité
f) au moins une étape d'analyse des images pour reconnaître les positions géométriques des inhomogénéités et au moins une caractéristique observable de chacune des inhomogénéités reconnues par exemple taille, facteur de forme, type de particules, âge de la particule, densité de biomatériau, type de biomatériau, molécule et
g) au moins une étape de sélection de l'une des inhomogénéités ainsi localisée et **caractérisée**, et
h) au moins une étape de transfert comprenant une commande du moyen de déviation pour diriger le faisceau laser vers la position de l inhomogénéité et une commande de déclenchement d'un tir laser.

14. Procédé pour le transfert de bioencre sur une cible selon la revendication 13 **caractérisé en ce que** les inhomogénéités à transférer sont une combinaison de particules, et/ou biomatériaux et/ou espèces chimiques.

15. Procédé pour le transfert de bioencre sur une cible selon la revendication 13 **caractérisé en ce qu'**il comporte une étape d'imagerie, d'analyse et de caractérisation et une pluralité d'étapes de transfert correspondant chacune à au moins une inhomogénéité localisée et **caractérisée**.

16. Procédé pour le transfert de bioencre sur une cible selon la revendication 13 **caractérisé en ce qu'**il comporte une étape de calcul d'une séquence ordonnée d'inhomogénéités à transférer.

17. Procédé pour le transfert de bioencre sur une cible selon la revendication précédente **caractérisé en ce qu'**il comporte une étape additionnelle de déclenchement d'un nouveau transfert pour corriger la différence entre le transfert observé et l'implantation théorique.

## Patentansprüche

1. Ausrüstung zum Übertragen von Biotinte auf ein Ziel, umfassend:
- einen transparenten Objektträger im Spektralbereich der Abbildung und des Lasers (5), der einen Bereich zum Aufnehmen eines Films (3) aus Flüssigkeit mit Heterogenitäten definiert,
- eine Laserquelle (5), die einem geführten Ablenkmittel und einer optischen Einheit (10) zur Bündelung in einer Ebene des Films (3) aus Flüssigkeit zum Anlegen eines lokalisierten Impulses zugeordnet ist, **dadurch gekennzeichnet, dass** die Ausrüstung ferner umfasst:
- ein Mittel zum Abbilden eines Abbildungsbereichs mit einem Abbildungsabschnitt, der mindestens fünfmal größer ist als der nominelle Abschnitt einer Inhomogenität,
- Bildanalysemittel zum Erkennen
• der geometrischen Positionen der Inhomogenitäten im Film, und
• eines beobachtbaren Merkmals jeder der erkannten Inhomogenitäten, zum Beispiel Größe, Formfaktor, Partikeltyp, Alter des Partikels, Dichte, Art des Biomaterials, Molekül, durch geeignete Analysemittel, und
- Mittel zum Auswählen mindestens eines der so lokalisierten und charakterisierten inhomogenen Bereiche und zum Steuern des Ablenkmittels, um den Laserstrahl in Richtung der Position des inhomogenen Bereichs zu lenken und den Laserschuss auszulösen.

2. Ausrüstung zum Übertragen von Biotinte auf ein Ziel nach Anspruch 1, **dadurch gekennzeichnet, dass** die Inhomogenität der Biotinte aus Partikeln besteht.

3. Ausrüstung zum Übertragen von Biotinte auf ein Ziel nach Anspruch 1, **dadurch gekennzeichnet, dass** die Inhomogenität der Biotinte aus Biomaterialien besteht.

4. Ausrüstung zum Übertragen von Biotinte auf ein Ziel nach Anspruch 1, **dadurch gekennzeichnet, dass** die Inhomogenität der Biotinte aus biochemischen Spezies besteht.

5. Ausrüstung zum Übertragen von Biotinte auf ein Ziel nach Anspruch 1, **dadurch gekennzeichnet, dass** die Inhomogenität der Biotinte aus einer Kombination von Partikeln und/oder Biomaterialien und/oder biochemischen Spezies besteht.

6. Ausrüstung zum Übertragen von Biotinte auf ein Ziel nach Anspruch 1, **dadurch gekennzeichnet, dass** der Abbildungsbereich den Wechselwirkungsbereich des Lasers mit dem Flüssigkeitsfilm umfasst.

7. Ausrüstung zum Übertragen von Biotinte auf ein Ziel nach Anspruch 1, **dadurch gekennzeichnet, dass** sich der Abbildungsbereich von dem Wechselwirkungsbereich des Lasers mit dem Flüssigkeitsfilm unterscheidet.

8. Ausrüstung zum Übertragen von Biotinte auf ein Ziel nach Anspruch 1, **dadurch gekennzeichnet, dass** sie ein lokales Rechengerät umfasst, das Bildverarbeitungen zur Charakterisierung der im Abbildungsbereich vorhandenen Inhomogenitäten ausführt.

9. Ausrüstung zum Übertragen von Biotinte auf ein Ziel nach Anspruch 1, **dadurch gekennzeichnet, dass** sie Kommunikationsmittel mit einem entfernten Rechengerät umfasst, das Bildverarbeitungen zur Charakterisierung der im Abbildungsbereich vorhandenen Inhomogenitäten ausführt.

10. Ausrüstung nach Anspruch 1, **dadurch gekennzeichnet, dass** der Film lebende Zellen enthält.

11. Ausrüstung nach Anspruch 1, **dadurch gekennzeichnet, dass** sie mehrere Laser-Materie-Wechselwirkungsbereiche umfasst.

12. Ausrüstung nach Anspruch 1, **dadurch gekennzeichnet, dass** sie ein Saugsystem zum Entfernen überschüssiger Druckelemente aus dem Druckbereich umfasst.

13. Verfahren zum Übertragen von Biotinte auf ein Ziel, bestehend aus dem Anordnen eines transparenten Objektträgers in einer Übertragungsausrüstung, der einen Bereich zum Aufnehmen eines transparenten Flüssigkeitsfilms definiert, der mehrere Inhomogenitäten enthält, und dem Steuern der Ausrichtung und des Auslösens eines Laserimpulses, der von einem Laser emittiert wird, der mit einem geführten Ablenkmittel verbunden ist, um eine Wechselwirkung des Laserstrahls mit dem Film zu bewirken, **dadurch gekennzeichnet, dass** das Verfahren ferner umfasst:
e) mindestens einen Schritt des Abbildens eines Bereichs des Films mit einem Abbildungsabschnitt, der mindestens fünfmal größer ist als der nominelle Abschnitt einer Inhomogenität,
f) mindestens einen Schritt des Analysieren der Bilder, um die geometrischen Positionen der Inhomogenitäten und mindestens ein beobachtbares Merkmal jeder der erkannten Inhomogenitäten zu erkennen, zum Beispiel Größe, Formfaktor, Partikeltyp, Alter des Partikels, Dichte des Biomaterials, Art des Biomaterials, Molekül, und
g) mindestens einen Schritt des Auswählens einer der so lokalisierten und charakterisierten Inhomogenitäten, und
h) mindestens einen Schritt des Übertragens, umfassend eine Steuerung des Ablenkmittels zum Richten des Laserstrahls auf die Position der Inhomogenität und eine Steuerung zum Auslösen eines Laserschusses.

14. Verfahren zum Übertragen von Biotinte auf ein Ziel nach Anspruch 13, **dadurch gekennzeichnet, dass** die zu übertragenden Inhomogenitäten eine Kombination von Partikeln und/oder Biomaterialien und/oder chemischen Spezies sind.

15. Verfahren zum Übertragen von Biotinte auf ein Ziel nach Anspruch 13, **dadurch gekennzeichnet, dass** es einen Abbildungs-, Analyse- und Charakterisierungsschritt und mehrere Übertragungsschritte umfasst, die jeweils mindestens einer lokalisierten und charakterisierten Inhomogenität entsprechen.

16. Verfahren zum Übertragen von Biotinte auf ein Ziel nach Anspruch 13, **dadurch gekennzeichnet, dass** es einen Schritt zum Berechnen einer geordneten Folge von zu übertragenden Inhomogenitäten umfasst.

17. Verfahren zum Übertragen von Biotinte auf ein Ziel nach dem vorhergehenden Anspruch, **dadurch gekennzeichnet, dass** es einen zusätzlichen Schritt des Auslösens einer neuen Übertragung umfasst, um den Unterschied zwischen der beobachteten Übertragung und der theoretischen Implantation zu korrigieren.

## Claims

1. Apparatus for transferring bio-ink to a target, comprising:
- a slide that is transparent in the spectral range of the imaging and of the laser (5), which slide defines an area for receiving a fluid film (3) containing heterogeneities;
- a laser source (5) associated with a controlled deflection means and an optical system (10) for focusing in a plane of the fluid film (3) in order to apply a localized pulse,
**characterized in that** said apparatus further comprises:
- means for imaging an imaging area having an imaging cross section at least 5 times greater than the nominal cross section of an inhomogeneity;
- means for analyzing images in order to recognize
• the geometric positions of the inhomogeneities in the film, and
• an observable characteristic of each of the recognized inhomogeneities, for example size, aspect ratio, type of particles, age of the particle, density, type of biomaterial or molecule, that can be observed by appropriate analysis means; and
- means for selecting at least one of the inhomogeneous areas thus located and characterized, and for controlling the deflection means in order to direct the laser beam toward the position of said inhomogeneous area and to trigger the laser shot.

2. Apparatus for transferring bio-ink to a target according to claim 1, **characterized in that** the inhomogeneity of said bio-ink consists of particles.

3. Apparatus for transferring bio-ink to a target according to claim 1, **characterized in that** the inhomogeneity of said bio-ink consists of biomaterials.

4. Apparatus for transferring bio-ink to a target according to claim 1, **characterized in that** the inhomogeneity of said bio-ink consists of biochemical species.

5. Apparatus for transferring bio-ink to a target according to claim 1, **characterized in that** the inhomogeneity of said bio-ink consists of a combination of particles and/or biomaterials and/or biochemical species.

6. Apparatus for transferring bio-ink to a target according to claim 1, **characterized in that** said imaging area includes the area of interaction between the laser and the fluid film.

7. Apparatus for transferring bio-ink to a target according to claim 1, **characterized in that** said imaging area is separate from the area of interaction between the laser and the fluid film.

8. Apparatus for transferring bio-ink to a target according to claim 1, **characterized in that** it comprises a local computer that performs image processing for the characterization of the inhomogeneities present in the imaging area.

9. Apparatus for transferring bio-ink to a target according to claim 1, **characterized in that** it comprises means for communicating with a remote computer that performs image processing for the characterization of the inhomogeneities present in the imaging area.

10. Apparatus according to claim 1, **characterized in that** said film contains living cells.

11. Apparatus according to claim 1, **characterized in that** it comprises a plurality of areas of laser-material interaction.

12. Apparatus according to claim 1, **characterized in that** it comprises a suction system for removing excess printed elements from the printing area.

13. Method for transferring bio-ink to a target, comprising arranging, in a transfer apparatus, a transparent slide that defines an area for receiving a transparent fluid film containing a plurality of inhomogeneities, and controlling the orientation and triggering of a laser pulse that is emitted by a laser and is associated with a controlled deflection means, in order to cause interaction between the laser beam and said film, **characterized in that** the method further comprises:
e) at least one step of imaging an area of the film having an imaging cross section at least 5 times greater than the nominal cross section of an inhomogeneity;
f) at least one step of analyzing images in order to recognize the geometric positions of the inhomogeneities and at least one observable characteristic of each of the recognized inhomogeneities, for example size, aspect ratio, type of particles, age of the particle, density of biomaterial, type of biomaterial or molecule; and
g) at least one step of selecting one of the inhomogeneities thus located and characterized; and
h) at least one transfer step including controlling the deflection means in order to direct the laser beam toward the position of one inhomogeneity and controlling triggering of a laser shot.

14. Method for transferring bio-ink to a target according to claim 13, **characterized in that** the inhomogeneities to be transferred are a combination of particles and/or biomaterials and/or chemical species.

15. Method for transferring bio-ink to a target according to claim 13, **characterized in that** it comprises an imaging, analysis and characterization step and a plurality of transfer steps each corresponding to at least one located and characterized inhomogeneity.

16. Method for transferring bio-ink to a target according to claim 13, **characterized in that** it comprises a step of calculating an ordered sequence of inhomogeneities to be transferred.

17. Method for transferring bio-ink to a target according to the preceding claim, **characterized in that** it comprises an additional step of triggering a further transfer in order to correct the difference between the observed transfer and the theoretical implantation.
